# EUROPEAN PATENT APPLICATION

(11) **EP 2 522 359 A1**
(43) Date of publication of application: **14.11.2012**
(21) Application number: 11305553.7
(22) Date of filing: 09.05.2011
(51) Int. Cl.: A61K 38/44, A61P 25/00

(54) **Compounds with thioredoxin-like fold domain for the treatment of degenerative disorders**

(71) Applicant: UNIVERSITE DE ROUEN, 76130 Mont-Saint-Aignan (FR)
(72) Inventor: Anouar, Youssef, 76230, Quincampoix (FR); Tanguy, Yannick, 76250, DEVILLE LES ROUEN (FR); Falluel-Morel, Anthony, 76150, LA VAUPALIERE (FR)
(74) Representative: Cabinet Plasseraud

(57) **Abstract**

The invention relates to compounds comprising at least one thioredoxin-like fold domain (CXXU) for use as medicament, particularly for use for the treatment and/or the prevention of degenerative disorders.

## Description

### FIELD OF THE INVENTION

The invention relates to compounds comprising at least one thioredoxin-like fold domain for the treatment and/or the prevention of degenerative disorders.

### BACKGROUND OF THE INVENTION

Degenerative disorders, also called neurodegenerative disorders, like Parkinson's disease, Alzheimer's disease, Huntington's disease, multiple sclerosis or stroke, are characterized by neurodegenerative changes or neuron apoptosis, which lead to brain dysfunction. Said disorders involve many factors, like protein abnormalities (mutations, misfolding...) and the formation of reactive oxygen species as a consequence of oxidative stress. Said disorders may be categorized according to the specific location and cell type involved.

Alzheimer's disease affects essential neurons involved in cognitive functions, and localized in different regions of the brain, including hippocampus and cortex. In disease forms due to mutations, proteins such as β-amyloid protein abnormally accumulate in these neurons.

Parkinson's disease affects dopaminergic neurons of the substantia nigra of the midbrain. The first symptoms are movement-related, and later cognitive troubles may arise.

Huntington's disease is a genetic neurodegenerative disease, which affects muscle coordination and later cognition. It manifests by abnormal involuntary writhing movements called chorea.

Multiple sclerosis, also called disseminated sclerosis or encephalomyelitis disseminata, is an inflammatory disease in which demyelination of the axon sheaths occurs. This leads to an alteration in the communication between neurons of the brain and spinal cord, and then to cognition and physical disability.

Stroke, also called cerebrovascular accident, is the rapidly developing loss of brain function due to a lack of blood supply to the brain. The resulting affected brain area is unable to function correctly, and this leads to physical invalidity and cognition troubles.

Today, there is no efficient treatment for most of the neurodegenerative disorders. The main treatments are symptomatic in nature, and may delay the progression of the disorder, but do neither prevent nor cure said disorder.

There is thus an important need for an efficient treatment of neurodegenerative disorders.

The inventors showed for the first time that selenoprotein T, which is a compound comprising a thioredoxin-like fold domain, efficiently protects cells against oxidative stress. Indeed, it significantly enhances cell survival in response to oxidative stress. Thus, it would be an efficient treatment for degenerative diseases.

### SUMMARY OF THE INVENTION

The invention relates to compounds comprising at least one thioredoxin-like fold domain for use as medicament. The invention also relates to compounds comprising at least one thioredoxin-like fold domain for use for treating and/or preventing degenerative disorders (i.e. neurodegenerative disorders).

Examples of degenerative disorders are neurodegenerative disorders like Parkinson's disease, Alzheimer's disease, Huntington's disease, multiple sclerosis, stroke (i.e. cerebrovascular accident), motoneuron-associated diseases (i.e. Charcot disease), epileptic seizures or neurotoxicant-associated diseases.

The invention also relates to a method for treating and/or preventing a degenerative disorder in a subject comprising administering to the subject a therapeutically effective amount of a pharmaceutical composition comprising a compound comprising at least one thioredoxin-like fold domain.

The invention also relates to the use of a compound comprising at least one thioredoxin-like fold domain for the manufacture of a medicament for preventing and/or treating degenerative disorders.

### DETAILED DESCRIPTION OF THE INVENTION

### Definitions :

The term "thioredoxin-like fold domain" has its general meaning in the art and refers to the domain CXXU, in which C and U are cysteine and selenocysteine respectively, and X is an aminoacid. This domain is mainly found in enzymes that catalyze the formation of a disulfide bridge. The activity of said domain depends on the reductive properties of the cysteine and the selenocysteine. Said thioredoxin-like fold domain is typically flanked by a β-sheet on one side and an α-helix on the other side, giving rise to a -β-CXXU-α-domain.

Preferably, said thioredoxin-like fold domain is CX₁X₂U, wherein X₁ is chosen from glycine (G), threonine (T) and valine (V), and X₂ is chosen from alanine (A), leucine (L) and serine (S).

The expression "compound comprising a thioredoxin-like fold domain" means a compound comprising a CXXU domain which is functional. By functional, it is meant that the thioredoxin-like fold domain (CXXU) holds its biological activity, i.e. reducing other proteins by cysteine thiol-disulfide exchange. Said compound is preferably a peptide or a protein. Typically, a peptide comprises 6 to 20 aminoacids, whereas a protein comprises at least 21 aminoacids. Preferably said compound is chosen from selenoproteins and their fragments, more preferably human selenoproteins and their fragments.

Most preferably, said compound is chosen from human selenoprotein W (hSeIW), human selenoprotein V (hSelV), human selenoprotein H (hSelH), human selenoprotein T (hSelT) and their fragments.

By "fragments", it is meant fragments comprising a CXXU domain. Said fragments may comprise 4 to 1000 aminoacids, preferably from 6 to 100 aminoacids, preferably from 6 to 20 aminoacids.

Preferably, the compound comprising a CXXU domain is human SelT or one of its fragments.

As used herein, the term "subject" denotes a mammal, such as a rodent, a feline, a canine, and a primate. Preferably, a subject according to the invention is a human.

In its broadest meaning, the term "treating" or "treatment" refers to reversing, alleviating, inhibiting the progress of the disorder or condition to which such term applies, or one or more symptoms of such disorder or condition.

In its broadest meaning, the term "preventing" means delaying or inhibiting the onset of the disorder or condition to which such term applies, or one or more symptoms of such disorder or condition.

"Degenerative disorders" are also called neurodegenerative disorders. In said disorders, the function or the structure of the affected tissues or organs will progressively deteriorate over time. Degenerative disorders involve the progressive loss of structure or function of neurons, including neuron death. Neurodegenerative disorders include Parkinson's disease, Alzheimer's disease, Huntington's disease, multiple sclerosis, stroke, motoneuron-associated diseases (i.e. Charcot disease), epileptic seizures and neurotoxicant-associated diseases.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a nontoxic solid, semi-solid or liquid filler, diluent, encapsulating material or formulation auxiliary of any type.

By "biocompatible" is meant a material which elicits no or minimal negative tissue reaction including e. g. thrombus formation and/or inflammation.

### Therapeutic methods and uses

The present invention provides methods and compositions (such as pharmaceutical compositions) for preventing or treating degenerative disorders.

Thus, an object of the invention is a compound comprising at least one thioredoxin-like fold domain for use as medicament. Another object of the invention is a compound comprising at least one thioredoxin-like fold domain for use for treating and/or preventing degenerative disorders. The compound comprising at least one thioredoxin-like fold domain may be used for the treatment and/or the prevention of degenerative disorders like Parkinson's disease, Alzheimer's disease, Huntington's disease, multiple sclerosis, stroke, motoneuron-associated diseases (i.e. Charcot disease), epileptic seizures or neurotoxicant-associated diseases.

In a preferred embodiment, the compound comprising at least one thioredoxin-like fold domain is chosen from proteins. Preferably, said compound is chosen from selenoproteins and their fragments, preferably human selenoproteins and their fragments.

More preferably, said compound comprises a thioredoxin-like fold domain chosen from CGAU (SEQ ID NO:1), CGLU (SEQ ID NO:2), CTSU (SEQ ID NO:3) and CVSU (SEQ ID NO:4). Preferably, said compound comprises CVSU (SEQ ID NO:4).

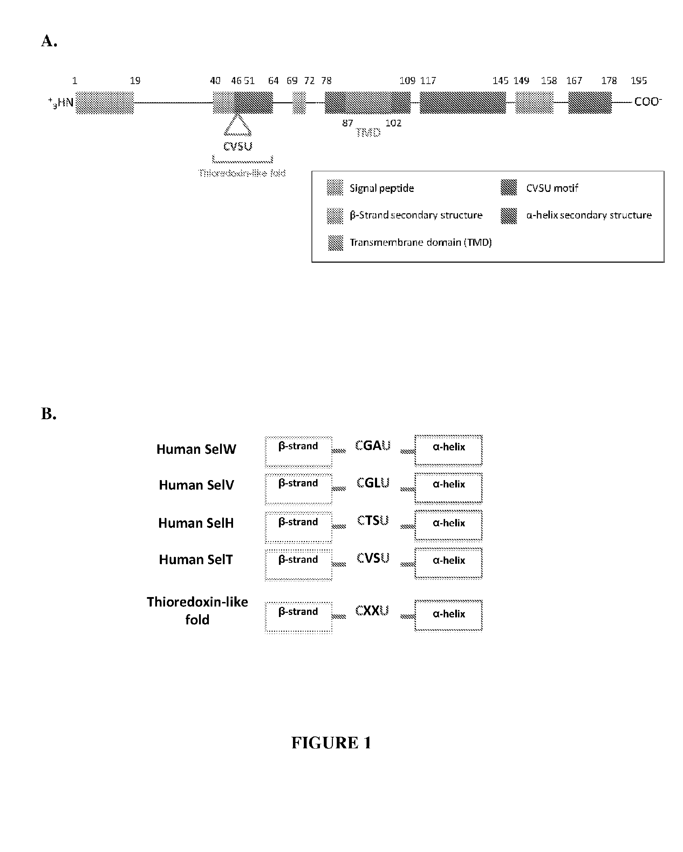

More preferably, said compound is chosen from selenoprotein W (SelW), selenoprotein V (SelV), selenoprotein H (SelH), selenoprotein T (SelT) and their fragments, preferably from SelT and its fragments. Most preferably, said compound is chosen from human selenoprotein W (hSelW), human selenoprotein V (hSelV), human selenoprotein H (hSelH), human selenoprotein T (hSelT) and their fragments. As shown in Figure 1, said hSelV, hSelH, hSelT and hSelW are homologous and comprise a thioredoxin-like fold domain.

Particularly, said compound is chosen from human selenoprotein T and its fragments.

Preferably, hSelT fragments comprise at least CVSU (SEQ ID NO:4).

Most preferably, hSelT fragments are chosen from CVSU (SEQ ID NO:4), ICVSU (SEQ ID NO:5), CVSUG (SEQ ID NO:6), QICVSU (SEQ ID NO:7), QICVSUG (SEQ ID NO:8), CVSUGY (SEQ ID NO:9), ICVSUGY (SEQ ID NO:10), QICVSUGY (SEQ ID NO:11), FQICVSU (SEQ ID NO:12), FQICVSUG (SEQ ID NO:13), FQICVSUGY (SEQ ID NO:14), CVSUGYR (SEQ ID NO:15), ICVSUGYR (SEQ ID NO:16), QICVSUGYR (SEQ ID NO:17), FQICVSUGYR (SEQ ID NO:18), KFQICVSU (SEQ ID NO:19), KFQICVSUG (SEQ ID NO:20), KFQICVSUGY (SEQ ID NO:21), KFQICVSUGYR (SEQ ID NO:22), CVSUGYRR (SEQ ID NO:23), ICVSUGYRR (SEQ ID NO:24), QICVSUGYRR (SEQ ID NO:25), FQICVSUGYRR (SEQ ID NO:26), KFQICVSUGYRR (SEQ ID NO:27) and (X)ₙ-KFQICVSUGYRR-(X')ₙ' in which X and X' represent any amino acid, and n and n' are integers between 1 and 1000.

The compound comprising at least one thioredoxin-like fold domain according to the invention may be chemically modified in order to maximize its stability or its efficiency, whether in its administration form or in vivo.

These chemical modifications concern any addition of a chemical group, like amide, alkyl or glucosidic groups, lipidic groups or natural or synthetic aminoacids or peptides.

Alkyl groups may be chosen from methyl and ethyl groups.

Glucosidic groups may be chosen from glucosides, like sinigrin, sinalbin, jalapin (or scammonin), arbutin, salicin, coniferin, syringin, aesculin, daphnin, fraxin, quercitrin, rhamnetin, saponarin, strophanthin and amygdalin.

Lipidic groups may be chosen from fatty acyls, glycerophospholipids and phospholipids.

Natural or synthetic amino acids or peptides may be chosen from those allowing to cross plasma membrane, such as PIP (Penetratin Internalization Peptide).

The compound comprising at least one thioredoxin-like fold domain may be administered in the form of a pharmaceutical composition, as defined below.

Preferably, said compound is administered in a therapeutically effective amount.

By a "therapeutically effective amount" is meant a sufficient amount of the compound comprising at least one thioredoxin-like fold domain, to treat and/or to prevent a degenerative disorder at a reasonable benefit/risk ratio applicable to any medical treatment.

It will be understood that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

### Pharmaceutical compositions:

A further object of the invention relates to a pharmaceutical composition for treating and/or preventing degenerative disorders such as Parkinson's disease, Alzheimer's disease, Huntington's disease, multiple sclerosis or stroke, wherein said composition comprises a compound comprising at least one thioredoxin-like fold domain.

The compound comprising at least one thioredoxin-like fold domain may be combined with a pharmaceutically acceptable excipient, and optionally a sustained-release matrix, such as biodegradable polymers, to form therapeutic compositions. Said compound may also be encapsulated in a suitable galenic form, like nanocapsules, beads, DNA vectors...

In the pharmaceutical compositions of the present invention for oral, sublingual, subcutaneous, intramuscular, intravenous, transdermal, nasal, pulmonary, mucosal, local or rectal administration, the active principle, alone or in combination with another active principle, can be administered in a unit administration form, as a mixture with conventional pharmaceutical supports, to animals and human beings. Suitable unit administration forms comprise oralroute forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal, intracerebroventricular and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, allow the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The compound comprising at least one thioredoxin-like fold domain of the invention can be formulated into a composition in a neutral or salt form. Pharmaceutically acceptable salts include the acid addition salts (formed with the free amino groups of the compound) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the compound comprising at least one thioredoxin-like fold domain, in the required amount, in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterilefiltered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermolysis fluid or injected at the proposed site of infusion. Some variation in dosage will necessarily occur depending on the condition of the subject being treated. The person responsible for administration will, in any event, determine the appropriate dose for the individual subject.

The compound comprising at least one thioredoxin-like fold domain of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds of the invention formulated for parenteral administration, such as intravenous or intramuscular injection, other pharmaceutically acceptable forms include, e.g. tablets or other solids for oral administration; liposomal formulations; time release capsules ; and any other form currently used.

Pharmaceutical compositions of the invention may include any further active agent which aims to prevent and/or treat a symptom of a degenerative disorder. Such active agents may include but are not limited to antibodies, receptor ligands, enzymes, oligonucleotides and the like. Such compounds may be antibodies (natalizumab...), immunomodulators (interferon beta-1b, mitoxantrone...), dopamine (L-DOPA), dopaminergic agonists (lisuride, ropinirol...), anticholinergics (amantadine...), NMDA (N-Methyl-D-Aspartate) antagonists (memantine hydrochloride...), cholinesterase inhibitors (donepezil or E2020, rivastigmine, galantamine bromhydrate...), tPA or alteplase. The foregoing substances are listed by way of example and are not meant to be limiting. Other active agents which are currently available or that may be developed in the future are equally applicable.

The invention will further be illustrated in view of the following figures and examples.

### FIGURES

**Figure 1****: Selenoprotein T secondary structure: highlight on its thioredoxin-like motif**
   Schematic illustration showing SelT functional domains.
   **A.** Bioinformatic analysis revealed the presence of α-helix and β-strand secondary structures and other domains such as a signal peptide, a thioredoxin-like domain, which contains the selenocystein residue (U), and a transmembrane domain (TMD) essential for its localization within the endoplasmic reticulum membrane.
   **B.** Thioredoxin-like family members, such as SelW, V, H and T, contain a particular motif corresponding to the thioredoxin-like fold (β-CXXU-α), where X designates any amino acid residue. This center, characterized by a strong redox activity, is considered as the active core of these selenoenzymes.

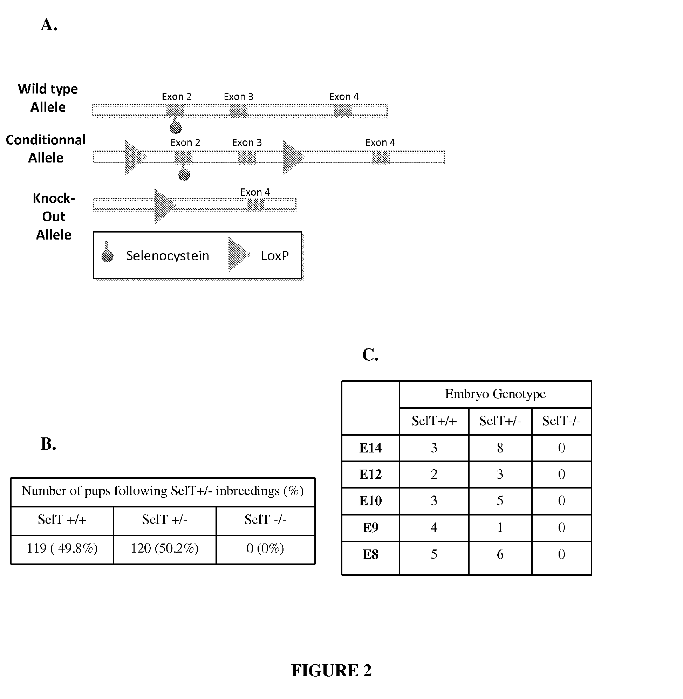

**Figure 2****: Embryonic lethality of Selenoprotein T-knockout mice**
   In order to elucidate selenoprotein T function, its genetic deletion was performed using the Cre/Lox technology.
   **A.** A mice strain carrying LoxP sequences flanking exons 2 and 3 (encoding the active core of the enzyme) was generated in collaboration with the Mouse Clinical Institute of Strasbourg. Genetic deletion was obtained by breeding these mice with a mice strain carrying a CMV-CRE recombinase allele. This method led to the generation of heterozygous animals presenting a knock-out allele.
   **B.** Genotyping performed on the second generation of pups obtained from heterozygous crossbreedings.
   **C.** Genotyping performed on embryos collected at several developmental stages. Embryos were removed from time-mated pregnant heterozygous females, which were crossed during three hours with heterozygous males.

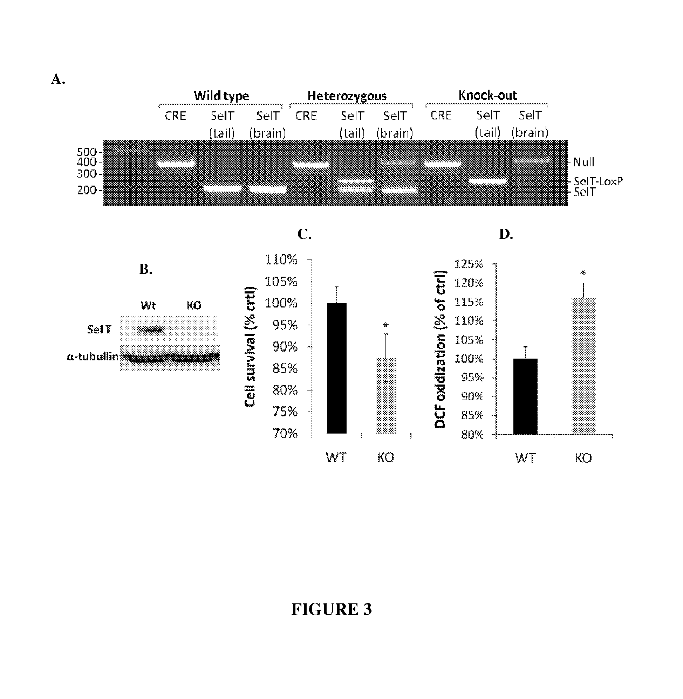

**Figure 3****: Selective brain knockout of Selenoprotein T gene in C57Black/6J mice**
   To circumvent embryonic lethality of SelT gene total deletion, a conditional knockout was generated by crossing SelT LoxP mice with animals expressing Cre recombinase selectively in the brain (nestin-Cre).
   **A.** PCR analysis of genomic DNA from a wild type, a heterozygous and a knockout animal. In the heterozygous and knockout animals, the SelTLoxP allele detected in the tail (non neural tissue) is recombined in the brain into a knockout allele, indicating that the selective deletion is successful.
   **B.** Western blot showing the absence of SelT protein in the brain of a newborn knockout animal. Alpha tubulin was used as a loading control.
   **C.** As a demonstration for SelT neuroprotective function during development, cell survival was investigated in cultured neurons obtained from E14.5 wild-type and knockout animals, 24 hours after plating. Results are expressed as means ±SEM of four animals per genotype.
   **D.** Comparison of endogenous ROS levels in wild-type and knockout E14.5 neurons, using DCF oxidization assay 24 hours after plating. Results are expressed as means ± SEM of four animals per genotype. *, P < 0.05 *vs.* control.

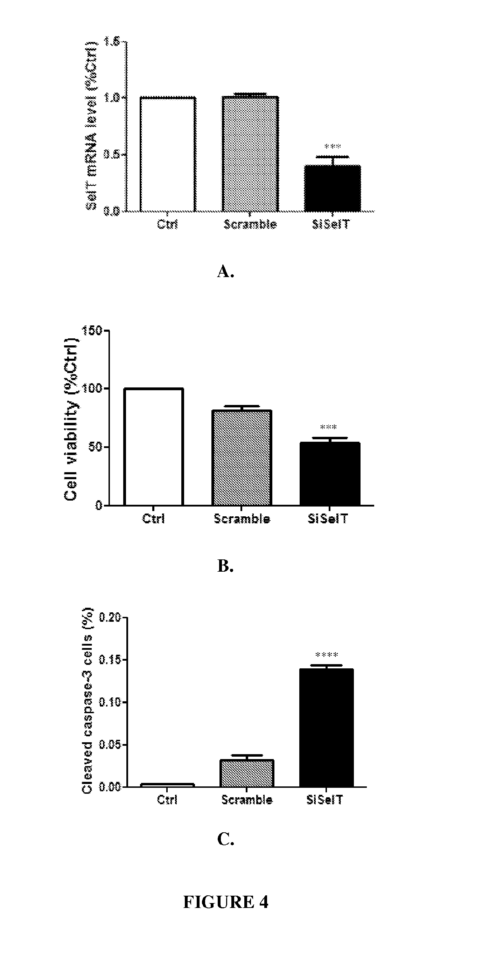

**Figure 4****: Effect of Selenoprotein T deficiency on astrocyte survival**
   In order to determine SelT importance in reactive astrocytes, its function was probed in cultured astrocytes that were transfected with SelT mRNA-targeting siRNA (siSelT, 100 nM). Control experiments were performed in similar conditions with non-transfected astrocytes (Ctrl) or astrocytes transfected with siRNAs that are unable to recognize any cellular RNA (Scramble).
   **A.** Quantitative PCR analysis of SelT mRNA levels in transfected astrocytes. Results are expressed as fold increase over corresponding control value, and represent means ± SEM of four independent experiments (one-way ANOVA; ***, *P <* 0.001 *vs.* the corresponding control).
   **B.** Cell viability was quantified with a FL600 fluorescence microplate reader after calcein incorporation in control, scramble and siRNA-transfected cells. Results are expressed as percentage of corresponding control value, and represent means ± SEM of four independent experiments (one-way ANOVA; ***, *P <* 0.001 *vs.* the corresponding control).
   **C.** The percentage of cleaved caspase-3-positive cells was calculated as the ratio of the number of cleaved-caspase-3-positive cells on the number of DAPI-stained total cells (1 000 cells counted for each condition). Values represent means ± SEM of four independent experiments and represented as percentages of control value (one-way ANOVA; ****, P < 0.0001 *vs.* the corresponding control).

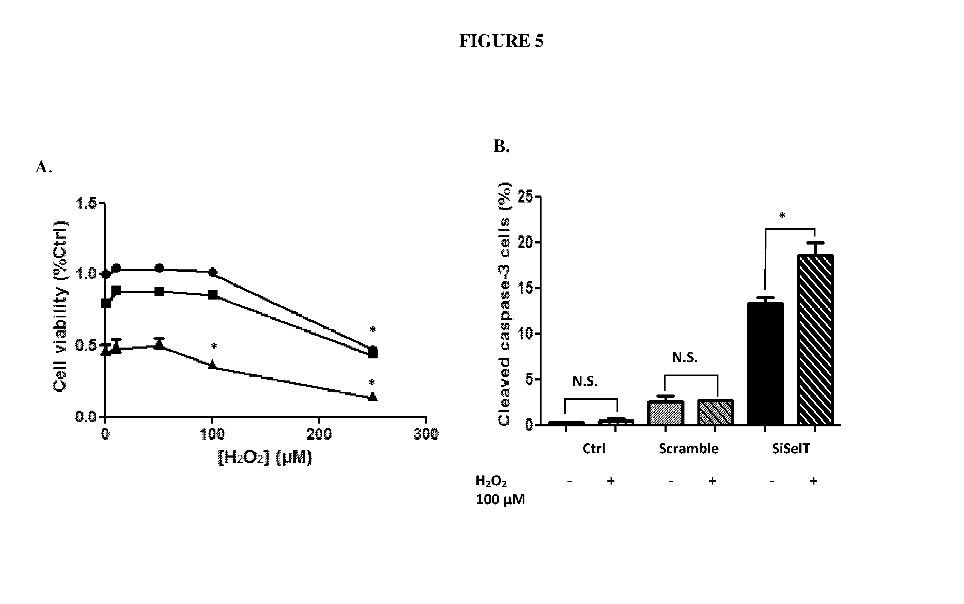

**Figure 5****: Effect of hydrogen peroxide on the survival of Selenoprotein T-deficient astrocytes**
   To emphasize SelT protective function against oxidative stress in astrocytes, control, scramble and siRNA-transfected cells were treated with H₂O₂.
   **A.** Viability of control and SelT-deficient astrocytes after 16 hrs-treatment with increasing concentrations of H₂O₂ (0-250 µM).
   **B.** Effect of H₂O₂ treatment (100 µM) on apoptosis induction in control and SelT-deficient astrocytes. Cleaved-caspase-3 was used as a marker for apoptotic cells, and was evaluated as previously described, by dividing the number of cleaved-caspase-3-positive cells by the total number of DAPI-stained cells. Results were obtained from three independent experiments, and are represented as means ± SEM (One-way ANOVA; *, P < 0.05).

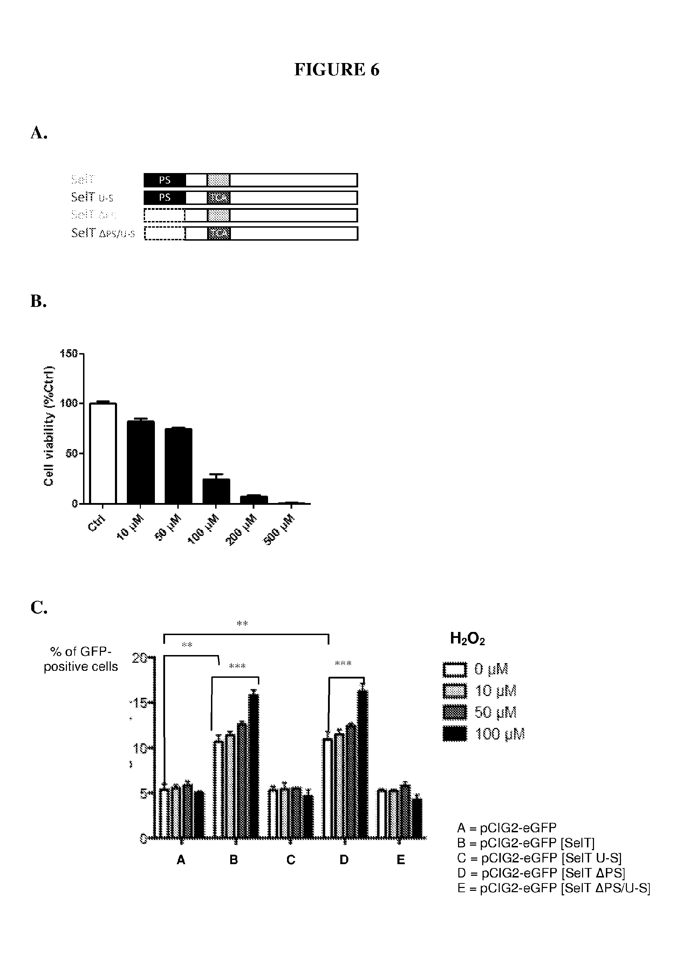

**Figure 6****: Neuroprotective effects of selenoprotein T : toward the proof of concept**
   This figure describes the strategy used to demonstrate the benefits provided by selenoprotein T and its selenium-containing active core on cell survival under normal and oxidative stress conditions. SelT and its mutated forms were incorporated in a pCiG2-eGFP vector in order to increase its levels following transfection in SH-SY-5Y neuroblastoma cell line.
   **A.** Representation of the four DNA constructs that were generated: Wild-type SelT was obtained by cloning the SelT cDNA isolated by PCR from rat PC12 cells. Using site-directed mutagenesis, we also prepared three mutant constructs that presented a serine-encoding codon (TCA) instead of the UGA-Selenocystein codon (SelT U-S) and/or a deletion of the sequence that encodes the signal peptide (ΔSP), in order to prevent the specific targeting of SelT to the endoplasmic reticulum.
   **B.** Dose-response of hydrogen peroxide on SH-SY-5Y cell survival. Results are represented as means ± SEM.
   **C.** Effect of increasing hydrogen peroxide concentrations (10, 50 and 100 µM) on survival of SH-SY-5Y cells transfected with either the empty pCiG2-eGFP vector or the same vector containing SelT or its mutated isoforms. Because the transfection process is efficient in only 5% of the cells, we are able to visualize in the same culture dish both untransfected and transfected cells (GFP positive). Thus, this paradigm allows us to study the effect of H₂O₂ treatment on the survival of both untransfected and transfected cells. Results are presented as the proportion of GFP-positive cells calculated from microphotographs acquired with a flurorescence microscope and are obtained by dividing green fluorescent cell number by total cell number. With pCiG2-eGFP or Sec-mutated SelT isoforms, the proportion of transfected cells is not influenced by hydrogen peroxide, indicating that sensitivity to oxidative stress is unaffected by the transfection of theses constructs. However, the percent of GFP-positive cells increases significantly following H₂O₂ treatment when cells are transfected with pCiG2-eGFP[SelT] or pCiG2-eGFP[SelTΔPS], thus demonstrating that cells that overexpress SelT (with or without the signal peptide) gain resistance to oxidative stress, while untransfected cells remain vulnerable. Data are presented as means ± SEM (two-way ANOVA, **, p < 0.01; ***, p < 0.001).

### EXAMPLE

### Materials & Methods

### Animals

Cultured astrocytes were obtained from one day-old Wistar rats (Charles River Laboratories, L'Arbresle, France). Middle cerebral artery occlusion experiments were done on three-month-old male C57BL/6J mice (Charles River). For neuroblast culture experiments, embryos were surgically removed from time-mated pregnant heterozygous (SelTLoxP / Nes-Cre) C57BL/6J females. Animals were housed under standard and constant conditions, with controlled lighting (12/12 hours light/dark cycle) and temperature (22°C ^{+/- 2°C}) and allowed access to food and water ad libitum. All experiments were conducted in accordance with the European Committee Council Directive and approved by the Regional Animal Experiments Committee (Comité Régional d'Ethique en Experimentation Animale de Normandie, agreements # N/01-11-07/09/12-10 and # N/05-12-10/06/02-13). Furthermore, experiments on animals were carried out in the presence of authorized investigators.

SelT genetic invalidation was performed through the generation of a C57BL/6J mouse strain whose genome carries LoxP sites flanking exon 2 and 3. These exons encompass the putative UGA-containing active domain, whose targeted invalidation leads to a non-functional protein (1). These mice were generated in the MCI (Mouse Clinical Institute, Strasbourg, France). Briefly, a plasmid construct was designed that contains SelT exons 2 and 3 flanked by two LoxP sites and a neomycin selection cassette comprised between Frt sequences. This recombinant vector was injected in 129 SV ES cells (MCI-129Sv/Pas), and clones in which the transgene was correctly inserted in SelT gene locus by homologous recombination were selected by treatment with appropriate selection agents. Then, clones were injected in several C57BL/6 blastocysts and newborn chimeric mice were, at adulthood, bred with Flipoverexpressing C57Black/6J mice (75 % C57Black/6J and 25 % 129SV). This crossing allowed removal of neomycin gene and the direct generation of heterozygous animals (SelTLoxP/SelT), which were characterized by PCR with the following primers, SelT-LoxP-F, GGCTTTATGTAAGCAGTTCTAAACTGTTTCTGC (SEQ ID NO:28) and SElT-LoxP-R, CGCCCCATTTTATAAACTTTGTATGTTTATGCCC (SEQ ID NO:29). This couple of primers allows discriminating wild-type allele (211 bp amplicon size) versus the conditional allele (261 bp). Then heterozygous mice were finally bred with recombinase Cre-expressing mice (MCI). The genome of these animals presents a Cre trangene, whose expression is under the control of the ubiquitous CMV promoter, allowing deletion of SelT gene at the first embryogenic stages (Cre primer couple, CMV-Cre-F, ATCGCCAGGCGTTTTCTGAGCATAC (SEQ ID NO:30) and, CMV-Cre-R, GCCAGATTACGTATATCCTGGCAGC (SEQ ID NO:31)). Finally, the invalidated allele contained in the next generations was detected with the following primers, SelT-KO-F, GGCTTTATGTAAGCAGTTCTAAACTGTTTCTGC (SEQ ID NO:32) and SelT-KO-R, GCCTAGGTTTTACCTGAGAAACCAAAGG (SEQ ID NO:33). The generation of 409 bp-sized amplicons illustrates the narrowing between SelT introns 1 and 4. The animals were housed in similar conditions as previously described. In order to determine embryo genotype at diverse and precise developmental stages, *primipara* heterozygous females were bred with heterozygous males during 3 hours and pregnancy was controlled by checking female weight (an increase by 10% of the body weight 8 days after breeding in addition with genital plug checking are key elements to determine pregnancy).

In parallel, female heterozygous for the floxed SelT allele (100% of genetic background purity) were bred with transgenic mice that express Cre recombinase under the rat nestin promoter (Tg (Nes-Cre) 1 Kln ; Jackson laboratory, Bar Harbor, Maine, USA). This crossbreeding allowed selective excision of SelT exons 2 and 3 in nestin positive cells, which will differentiate into astrocytes and neurons. Genotypes were determined by PCR on tail and brain DNA with the primers described above.

### MCAO experiments

Young adult male C57BL6/J mice (3 month-old) were purchased from Charles River. Animals were anesthetized through an intraperitoneal injection of Ketamine (Imalgene 500, 100 mg/kgbw) and Xylazine (Rompun, 10 mg/kgbw), and temperature was monitored and maintained at 37°C during surgery. Unilateral MCAO of the right cerebral hemisphere was performed by electrocoagulation (Gregersen *et* a/., 2000) using an electrosurgery unit (Unit Martin ME102 ; Harvard Apparatus, Les Ulis, France). Sham animals were anesthetized and underwent the same surgical protocol except for electrocoagulation. After surgery, animals were rehydrated, and housed in a light and temperature controlled animal rack (26°C^{+/-1°C}). Moreover, each mouse which presented signs of pain or illness was immediately euthanatized. Animals were anesthetized and euthanized at various times following MCAO, and tissue sampling method was chosen depending on western blot or immunohistochemistry experiments. The efficiency of MCAO was checked by a triphenyltetrazolium chloride 0.1% coloration on 3 mm-thick brain slices. lmmunohistochemistry was performed on 40 µm-thick floating serial coronal sections cut with a vibratome (Leica Microsystems, Nanterre, France).

### Tissue processing

For western blot analysis, C57BL/6J mice were anesthetized by isofluran inhalation (AErrane®, Baxter, Maurepas, France) and then immediately euthanized by decapitation with a guillotine for rodents (Harvard Apparatus). Then, sampled tissues were immediately frozen. lmmunohistochemistry experiments required to deeply anesthetize animals with an intraperitoneal sodium pentobarbital injection (120 mg/kgbw, Ceva Santé Animale, Libourne, France), and were transcardially perfused with a saline solution (0.9 % NaCl), followed by 4% paraformaldehyde in 0.1 M PBS pH 7.4. Tissues were removed and postfixed in the same fixative solution overnight at 4°C. Tissues were finally sectioned on a vibratome (thickness 40 µm ; Leica) and sections were conserved in PBS containing 0.1 % sodium azide.

### Plasmid constructions

Total RNAs were isolated using the Tri-Reagent (Sigma-Aldrich) and reverse-transcribed using the ImProm-II ReverseTranscription System (Promega, Charbonnières, France). Forward and reverse primers to amplify the full length cDNA of rat SelT including the SECIS domain were designed according to the sequences of the rat SelT and were terminated at the 5' and 3' ends by EcoRl and Xhol restriction sites, respectively, as follows: 5'-GAATTCGCCAGCATGGAAAACTGTCT-3' (SEQ ID NO:34) and 5'-CTCGAGGGGCTGCAGTCTCGGTCTGAAG-3' (SEQ ID NO:35). Other primers were designed and used in order to amplify an N-terminally truncated form of rat SelT, SelTΔPS-F, AGCGGCTGTCTCAATTAAGATGTCTGCCAACCTGGGCGGCGTGCCCA (SEQ ID NO:36), SelTΔPS-R, TGGGCACGCCGCCCAGGTTGGCAGACATCTTAATTGAGACAGCCGCT (SEQ ID NO:37), and SelT that contained a TGA codon mutated into a TCA signal that encodes a serine instead of Sec, SelT-US-F, TTTCAGATTTGTGTATCCTCAGGGTACAGGCGGGTGTTT (SEQ ID NO:38); SelT-US-R, AAACACCCGCCTGTACCCTGAGGATACACAAATCTGAAA (SEQ ID NO:39). PCR was performed using a Stratagene's QuikChange® site-directed mutagenesis (Agilent, Massy, France) in Perkin-Elmer GeneAmp PCR system 9700 (Applied Biosystems), and the products were ligated into the pClG2-eGFP vector. Sequencing was performed with a Beckman seq 8 000 sequencer (Beckman Coulter, Villepinte, France).

### Cortical neuron culture

Dorso-lateral cerebral cortices were removed from 15 day-pregnant heterozygous females (SelT-LoxP/SelT ; Nes-Cre), separated from basal ganglia and overlying meninges. Cells were mechanically dissociated in the culture medium (Neurobasal (Invitrogen) containing 50 U/mL penicillin (Sigma-Aldrich), 50 µg/mL streptomycin (Sigma-Aldrich), 1% BSA, 2 mM Glutamine (Invitrogen) and 2% B27 serum free supplement (Invitrogen). Cells were plated on 0.1 mg/mL poly-D-lysine and 5 µg/mL laminine coated culture dishes, and incubated at 37°C in 5% CO₂.

### Astrocyte culture

Primary cultures of cortical astrocytes were prepared as previously described (10). Cerebral hemispheres from newborn Wistar rats were collected in DMEM/Ham-F12 (2/1, v/v) culture medium supplemented with 2 mM glutamine (Invitrogen), 1% insulin (Sigma-Aldrich), 5 mM HEPES (Sigma-Aldrich), 0.4% glucose (Sigma-Aldrich) and 1% of an antibiotic-antimycotic solution (Sigma-Aldrich). The tissues were mechanically dissociated with a syringe equipped with a 1-mm gauge needle, and filtered through a 100-µm pore size mesh filter (Falcon, Becton Dickinson, Grenoble, France). Dissociated cells were resuspended in culture medium supplemented with 10% heat-inactivated FBS and seeded in 150-cm² culture flasks (Falcon) at a density of 20 × 10⁶ cells per flask. The cells were incubated at 37°C in a humidified atmosphere (5% CO₂) and the medium was replaced twice a week. When cultures reached confluence, the flasks were gently shaken on an orbital shaker at 250 rpm for 2 hrs. Suspended cells were discarded and a second step of purification was performed at 250 rpm for 14-16 hrs. Remaining adhesive cells were collected by trypsinization, centrifuged (800 rpm, 10 min) and plated in 150-cm² flasks. Nearly pure astrocytes were harvested and seeded in poly-L-lysine-coated microscopy coverslips in 24-well plates (for immunocytochemistry experiments) or in 12-well plates (for western-blot, RT-PCR and Live/Dead experiments). The purity of the cultures assessed by counting the percentage of astrocytes immunostained with GFAP antibodies was 99% (11).

Then, SelT silencing was performed using siRNA (Stealth RNAi™, Invitrogen). Two different molecules were designed and used at 100 nM ; siRNA-SelT1, ACA UAG CAU UUC UCC UGU CAG UCU U (SEQ ID NO:40) and siRNA-SelT2, AAG ACU GAC AGG AAA GAU GCU AUG U (SEQ ID NO:41). Transfection protocol with Lipofectamine™ 2000 (Invitrogen) was optimized with 4 µL of reagent in each well of a confluent 12 well-plate , incubated in a serum-free medium (DMEM supplemented with 2 mM glutamine), for 6 hours. Then, transfection medium was removed and replaced with regular serum-containing medium. After 48 hours, astrocytes were treated overnight with hydrogen peroxide (Sigma-Aldrich).

### Neuroblastoma SH SY-5Y culture

The human neuroblastoma SH-SY 5Y cell line was cultured as previously described in a Dulbecco's modified Eagle's medium (DMEM) supplemented with 10% heat inactivated fetal bovine serum (Sigma-Aldrich), 1% L-glutamine (Invitrogen), 1% HEPES (Sigma-Aldrich) and 0.1% of antibiotic-antimycotic solution (Sigma-Aldrich). Cells were plated in 12-well plates until they reached 95% confluence, and then were transfected with various pClG2 plasmid constructs using Lipofectamine™ 2000 (Invitrogen). Finally, transfected cells were analyzed with a fluorescence microscope (Leica, PRIMACEN).

### Western blot

Tissues were homogenized in a lysis buffer (Tris-HCl 50 mM, β-mercaptoethanol 2 mM, PMSF 2 mM, triton X-100 1 % and a mix of protease inhibitors (Roche, Mannheim, Germany)), proteins were quantified with Bradford method (BioRad, Marnes-la-Coquette, France) and then total proteins were precipitated with TCA 0.1 M. Precipates were washed and solubilized in Laemmli buffer (Tris-HCl 50 mM, SDS 0.68 M, β-mercaptoethanol 0.71 M, EDTA 1 mM, PMSF 2 mM, Glycerol 20 %, bromophenol blue 0.004 %). For all experiments, 50 µg of proteins were loaded on a 13 % SDS-PAGE, and immediately blotted onto a nitrocellulose membrane (Amersham Pharmacia Biotech, Les Ulis, France). Membranes were saturated with TBS-Tween containing 0.5 % BSA and 5 % fat-free dry milk, and SelT staining was carried out with a specific antibody generated in our laboratory diluted in a 5 % milk TBS-Tween 20 medium (1 : 2000) during one night at 4°C. Anti-α-tubulin antibody (Sigma-Aldrich) was used as internal loading control. Then, membranes were incubated with appropriate secondary HRP-horseradish antibodies during two hours at room temperature (Amersham Pharmacia Biotech). Signals were visualized with chemiluminescence (ECL, Amersham Pharmacia Biotech), and revealed on photographic film (Amersham Pharmacia Biotech).

### Immunohistochemistry

Injured brain tissue sections were saturated with 1 % donkey serum, diluted in a PBS-BSA buffer (PBS 0.1 M, BSA 1% and Triton X-100 0.3 %) for 45 minutes at room temperature, and exposed to SelT antibody (1:400) and GFAP (1:200, Santa Cruz Biotechnology, Bergheimer, Germany). After one night at 4°C, primary antibodies were detected through Alexa Fluor 488 and 594 secondary antibodies (1:200, Invitrogen, Cergy Pontoise, France). After 90 minutes at room temperature, a DAPI (Sigma-Aldrich) staining was performed (1 µg/mL in PBS, Sigma-Aldrich) for 90 seconds. Then, tissue sections were mounted with PBS-glycerol (50/50; v/v). The different labelings were examined through a Leica SP2 upright confocal laser scanning microscope (DMRAX-UV) equipped with the Acousto-Optico Beam Splitter system (Leica). This equipment was provided by the PRIMACEN imaging core facility (Mont Saint Aignan, France).

### Immunocytochemistry and cleaved caspase-3 staining

Cells were grown on coverslips and treated with 100 µM hydrogen peroxide. After one night of treatment, cells were fixed with 4% paraformaldehyde and immunolabeled with SelT or cleaved caspase-3. Cells were then incubated with Alexa Fluor 488-conjugated goat anti-rabbit lgG (Amersham Pharmacia Biotech) and counterstained with DAPI (Sigma-Aldrich). Cells were observed with a Leica SP2 upright confocal laser scanning microscope (Leica). For each experiment, 5 pictures/coverslip were taken randomly (4 coverslips per condition for each trial, 4 independent trials). The number of caspase-3 versus total cells was determined on each slide, thus allowing determining the proportion of apoptotic cells.

### RT reaction and quantitative PCR

Total RNA was isolated using the Tri-Reagent (Sigma-Aldrich) and reverse-transcribed using the ImProm-II Reverse Transcription System (Promega, Charbonnières, France). Gene expression quantification was performed by real-time PCR with the following primers, SelT-F, 5'-GGTCTAAGCTGGAATCTGGACATC-3' (SEQ ID NO:42), SelT-R, 5'-TGCACATTGAGTTTCATTTCATTG-3' (SEQ ID NO:43) (1) ; GAPDH-F, 5'-TCCGGACGCACCCTCAT-3' (SEQ ID NO:44), GAPDH-R, 5'-CGGTTGACCTCCAGGAAATC-3' (SEQ ID NO:45) ; SelW-F, 5'-TAGAGGCAGGGTCCTGAAG-3' (SEQ ID NO:46), SelW-R, 5'-AATCCATCTCTGGCCTGACT (SEQ ID NO:47) ; GRP78-F, GAAAGGATGGTCAATGACGCCGA (SEQ ID NO:48), GRP78-R, GTCTTCAATGTCT GCATCCTG (SEQ ID NO:49). PCR was carried out in SYBR Green PCR Master Mix™ (Applied Biosystems), with 300 nM of primers, using an ABI Prism 7500 (Applied Biosystems). Relative gene amplification efficiencies were assessed, and the amounts of targeted gene mRNA were determined using the comparative CT method (Applied Biosystems).

### Live Dead assay

Neuronal precursor or astrocyte survival was visualized after treatments using the LIVE/DEAD® Viability/Cytotoxicity Kit for mammalian cells (Invitrogen). Cells were washed with PBS and then incubated for 20 minutes with a PBS-solution containing 2 µM calcein and 4 µM ethidium homodimer. For quantification, fluorescence signals were measured with a FL600 fluorescence microplate reader using 485/530 and 485/590nm filters (Molecular Devices, St Grégoire, France). For illustration, images were obtained and examined using a fluorescent microscope (Leica DM HRBE-Metamorph, PRIMACEN).

### Measurement of intracellular ROS levels

Intracellular ROS levels were measured using H₂DCF (dihydro-dichlorofluorescein). This non-fluorescent compound reacts with ROS to generate a fluorescent product, the 2',7'-dichlorofluorescein (DCF) (12). Briefly, cells were washed with PBS and lysated into a cold lysis buffer (0.1 M phosphate buffer (pH 7.4), 140 mM KCI, 1 mM EDTA). After centrifugation, supernatant were exposed to the H₂DCF probe (1 µM) for 30 minutes at 37°C. DCF fluorescence was analyzed using a microplate reader (FL600, Biotek) with excitation and emission wavelengths of 488 and 525 nm, respectively. An aliquot of the lysate was used for protein quantification (Bradford assay, Biorad) in order to normalize results.

### Statistical analysis

All statistical analyses were performed with Prism 5 (GraphPad Prism. Inc., USA) using one way ANOVA analysis, followed by the Kruskal-Wallis post-test. *, P < 0.05; **, P < 0.01; ***, P < 0.001; ****, P <0.0001 *vs.* the corresponding control was considered to indicate significant statistical differences (not significant variation; N.S P > 0.05). Values represent means ± SEM.

### Results

### SelT function during embryonic development and neurogenesis

In previous studies, selenoprotein T expression and distribution was investigated in Wistar rat. As a prerequisite for the present work, mainly performed in C57black/6J mice, it was necessary to check that mice exhibit a similar pattern. Western blot analysis showed that, similarly to the rat brain, SelT is highly expressed during development, but its levels decrease significantly as the brain matures (data not shown). In the adult mouse brain, SelT expression is restricted to the olfactory bulb and the cerebellum (data not shown).

Consequently, as a strategy to identify the physiological function of selenoprotein T in normal or pathophysiological conditions, we carried out its genetic deletion in C57black/6J mice. In collaboration with the Mouse Clinical Institute of Strasbourg, we developed a mice strain whose genome was recombined so that it bears LoxP sites in the introns surrounding SelT catalytic domain (Fig. 2A). This particular region was chosen based on previous data indicating that the selenocytein-containing domain is crucial for SelT activity within the endoplasmic reticulum (1). We bred these SelT-LoxP mice with a second strain which express ubiquitously, and starting from the first embryonic stages, the recombinase cre, thus promoting the deletion of exons 2 and 3 (Fig. 2A). We successfully obtained heterozygous animals, identified by PCR performed on tail tip (data not shown); however, inbreeding failed to lead to the generation of homozygous pups (Fig. 2B). This result suggests that SelT invalidation is lethal *in utero* during the fetal period. Then, we tried to determine the fetal period when the homozygous embryos declined. To do so, embryos were surgically removed from time-mated *primipara* pregnant heterozygous females, and genotype was assessed with all or part of the embryo, depending on their size. Again, we did not identify any homozygous embryo from E8 to E14 (Fig. 2C). At earlier times than these mid-gestational stages, embryos were too small to be surgically removed. However, we tried to determine the genotype of E7 embryos excised from uterine frozen sections using laser microdissection. We identified several necrotic embryos, whose genotype was unclear due to the invasion by maternal-derived tissue (data not shown). Consequently, we deduced that the decline of homozygous embryos occurs during the first week of gestation, similarly to what was previously reported for Sec-tRNA (2) or PHGPx (3) knockout mice.

Because of a lack of SelT null animals, and because we previously demonstrated that SelT levels are particularly elevated in the developing brain (data not shown), we decided to generate brain-selective SeIT-/- transgenic animals. To do so, we bred SelT-LoxP animals with a mouse strain that carries a transgene encoding the recombinase Cre under the control of nestin promoter. This conditional knockout permitted to restrict the deletion of SelT gene in the neural cell lineage, which ultimately gives rise to neurons and astrocytes (4). Nestin-Cre mediated recombination begins around embryonic day 7.5, when the neural plate is being formed. It is assumed that recombination is complete in all neuronal and glial cells at E15 (5). Using this strategy, we obtained at the second generation live mice homozygous for SelT-LoxP and Nes-Cre(+). PCR was done on both nervous and non-nervous tissues to check the efficiency and specificity of Cre-mediated recombination (Fig. 3A). As expected, in the brain of mutant mice, SelT-LoxP allele recombined into the SelT-null form. In order to verify that the genetic recombination actually leads to the absence of SelT in the brain, a western blot analysis was performed on P1 mice brain. At this stage, while SelT expression is robust in wild-type animals, it is almost undetectable in the mutant (Fig. 3B), thus validating our knockout model. As an initial approach to investigate the potential neuroprotective function of SelT during brain development, we compared the survival of cortical neuroblasts obtained from wild-type and knockout E14.5 embryos (Fig. 3C). After one night in culture, knockout immature cortical neuron viability was altered, suggesting that SelT is involved in neuron survival mechanisms. In addition, we found that endogenous ROS levels are significantly higher in SelT-/- cells (Fig. 3D), suggesting that the alteration of their survival is related to a perturbation of the redox equilibrium. These data constitute the first piece of evidence for a neuroprotective function of SelT, which is essential during embryogenesis.

### SelT function in pathophysiological conditions: up-regulation in reactive astrocytes following stroke

Although SelT is abundant during embryogenesis and is poorly expressed in normal adult central nervous system, the possibility exists that its expression might be reinduced during pathophysiological conditions, especially after a cerebral stroke, as it was previously suggested (6). Consequently, we developed a model of a permanent ischemia through electrocoagulation of the middle cerebral artery in C57black/6J mice. Using this animal model, we explored SelT occurrence in injured hemispheres compared to sham animals. A western blot analysis indicated that SelT expression is rapidly and durably triggered after a cerebral ischemia (data not shown). A co-immunostaining of SelT with GFAP (Glial Acidic Fibrillary Protein) permitted to conclude that SelT is detected in reactive astrocytes, which proliferate at the periphery of the injured cortex (data not shown). Thus, these data reinforce the idea that SelT could play an important role in the control of cell survival, probably by joining the enzymatic arsenal involved in oxidative stress protection. To further test this hypothesis, we explored its function within cultured astrocytes, which were exposed to hydrogen peroxide as a mean to mimic an important oxidative stress. An immunoblot indicated that hydrogen peroxide treatment induces a concentration-dependant increase in SelT expression in glial cells (data not shown). Concurrently, to elicit the importance of SelT induction in these reactive cells, interfering stealth RNA, specifically designed to target SelT mRNA, were transfected in glial cells (Fig. 4). Twenty four hours after transfection, SelT mRNA levels (Fig. 4A) and protein immunoreactivity (data not shown) were both dramatically decreased with stealth RNA, when compared to control experiments performed on either non transfected cells or astrocytes transfected with stealth RNA that are unable to recognize any cellular RNA (scramble), thus validating the ability of this method to inhibit SelT expression. In these conditions, we were able to measure the impact or SelT invalidation on astrocyte viability (Fig. 4B). Thus, we observed that the deficit in SelT expression leads to astrocytic degeneration (Fig. 4, B and C). This increase in astrocyte death consecutive to SelT suppression is associated with an apoptotic process, as revealed by cleaved-caspase-3 immunostaining (Fig. 4C). Next, we examined the effect of hydrogen peroxide on astrocyte survival and apoptosis in the presence or absence of SelT (Fig. 5). Control and stealth RNA-transfected astrocytes were exposed overnight to increasing concentrations of hydrogen peroxide, and viability was assessed by measuring calcein incorporation. Although the survival profiles appeared very similar in the different conditions, we observed that the dose of 100 µM of hydrogen peroxide, which is not toxic on non transfected astrocytes (7), induced an additional 20% loss of SelT-deficient cells (Fig. 5A). Moreover, after incubating SelT-deficient glial cells with 100 µM H₂O₂, we observed that the proportion of caspase 3-positive cells also increased in a similar manner (Fig. 5B). In order to further investigate the degree of alteration of these cells, we next explored the impact of SelT invalidation on the expression of several stress-related factors, such as GRP78 or SelW (selenoprotein W), another selenoprotein sharing many similarities with SelT (8) which is up-regulated in SelT-deficient cells (9). In transiently transfected astrocytes, SelW does not compensate the lack of SelT, thus probably explaining why these cells are sensitive to oxidative stress. We noticed, however, that GRP78 mRNA levels, which are induced following H₂O₂ treatment in control cells, remained unchanged in SelT-deficient astrocytes (data not shown). This result suggests that these cells are unable to trigger the Unfolded Protein Response (UPR), an ER-stress related mechanism essential for cell survival and closely linked to oxidative insult. Taken together, these data indicate that SelT is essential for reactive astrocyte survival, especially during stress conditions that notably occur following cerebral stroke.

### Selenoprotein T overexpression enhances cell survival in normal and stress conditions

Although SelT has been implicated in calcium homeostasis regulation in our previous studies, nothing is known about its potential role in cell survival through its thioredoxin-like fold domain (Fig. 1). In order to perform gain and loss-of-function studies, we designed various SelT cDNA constructs that were introduced in a pCiG2 vector to allow their expression along with a GFP in SH-SY-5Y neuroblastoma cells. The functional domain was mutated through the replacement of the TGA selenocystein codon by a TCA signal that encodes a serine (ΔU-S; Fig. 6A), which has no redox properties but presents equivalent hydrophilicity and steric hindrance. We also generated a SelT isoform whose signal peptide was deleted in order to address the selenoprotein primarily in the cytosol (ΔPS; Fig. 6A). Prior to transfection, each cDNA construct was validated by sequencing (data not shown), and sensitivity of SH-SY-5Y cells to increasing concentrations of hydrogen peroxide was determined (Fig. 6B). Thus, cells transfected with either the empty pCiG2-eGFP vector or the same vector containing SelT or its mutated isoforms were exposed overnight to H₂O₂, and total cells and transfected cells expressing eGFP were counted. In untreated conditions, we observed that the proportion of transfected cells counted after 48 hours is significantly higher with constructs containing native or ΔPS SelT than with a pCiG2-eGFP or Sec-mutated SelT isoforms (Fig. 6C). This is probably due to the improvement of their survival abilities provided by SelT. Indeed, in the presence of SelT, GFP-evoked fluorescence appeared more intense and cells were free of vacuolar structures (data not shown). Moreover, while the total population declined following hydrogen peroxide treatment, the number of SelT-overexpressing cells remained stable, as illustrated by the significant increase in the percentage of eGFP-positive cells, suggesting that SelT strongly stimulated their viability under oxidative stress conditions. Finally, the deletion of SelT signal peptide did not alter its abilities to increase cell survival.

### REFERENCES

Throughout this application, various references describe the state of the art to which this invention pertains. The disclosures of these references are hereby incorporated by reference into the present disclosure.
1. Grumolato L, Ghzili H, Montero-Hadjadje M, Gasman S, Lesage J, Tanguy Y, Galas L, Ait-Ali D, Leprince J, Guerineau NC, Elkahloun AG, Fournier A, Vieau D, Vaudry H and Anouar Y (2008) Selenoprotein T is a PACAP-regulated gene involved in intracellular Ca2+ mobilization and neuroendocrine secretion. FASEB J. 22:1756-1768
2. Bosl MR, Takaku K, Oshima M, Nishimura S and Taketo MM (1997) Early embryonic lethality caused by targeted disruption of the mouse selenocysteine tRNA gene (Trsp). Proc. Natl. Acad. Sci. U. S. A 94:5531-5534
3. lmai H, Hirao F, Sakamoto T, Sekine K, Mizukura Y, Saito M, Kitamoto T, Hayasaka M, Hanaoka K and Nakagawa Y (2003) Early embryonic lethality caused by targeted disruption of the mouse PHGPx gene. Biochem. Biophys. Res. Commun. 305:278-286
4. Lendahl U, Zimmerman LB and McKay RD (1990) CNS stem cells express a new class of intermediate filament protein. Cell 60:585-595
5. Dubois NC, Hofmann D, Kaloulis K, Bishop JM and Trumpp A (2006) Nestin-Cre transgenic mouse line Nes-Cre1 mediates highly efficient Cre/loxP mediated recombination in the nervous system, kidney, and somite-derived tissues. Genesis. 44:355-360
6. lkematsu K, Tsuda R, Tsuruya S and Nakasono l (2007) Identification of novel genes expressed in hypoxic brain condition by fluorescence differential display. Forensic Sci. lnt. 169:168-172
7. Huang J and Philbert MA (1995) Distribution of glutathione and glutathione-related enzyme systems in mitochondria and cytosol of cultured cerebellar astrocytes and granule cells. Brain Res. 680:16-22
8. Dikiy A, Novoselov SV, Fomenko DE, Sengupta A, Carlson BA, Cerny RL, Ginalski K, Grishin NV, Hatfield DL and Gladyshev VN (2007) SelT, SelW, SelH, and Rdx12: genomics and molecular insights into the functions of selenoproteins of a novel thioredoxin-like family. Biochemistry 46:6871-6882
9. Sengupta A, Carlson BA, Labunskyy VM, Gladyshev VN and Hatfield DL (2009) Selenoprotein T deficiency alters cell adhesion and elevates selenoprotein W expression in murine fibroblast cells. Biochem. Cell Biol. 87:953-961
10. Gandolfo P, Patte C, Leprince J, Thoumas JL, Vaudry H and Tonon MC (1997) The stimulatory effect of the octadecaneuropeptide (ODN) on cytosolic Ca2+ in rat astrocytes is not mediated through classical benzodiazepine receptors. Eur. J. Pharmacol. 322:275-281
11. Castel H, Diallo M, Chatenet D, Leprince J, Desrues L, Schouft MT, Fontaine M, Dubessy C, Lihrmann I, Scalbert E, Malagon M, Vaudry H, Tonon MC and Gandolfo P (2006) Biochemical and functional characterization of high-affinity urotensin II receptors in rat cortical astrocytes. J. Neurochem. 99:582-595
12. LeBel CP, Ali SF, McKee M and Bondy SC (1990) Organometal-induced increases in oxygen reactive species: the potential of 2',7'-dichlorofluorescin diacetate as an index of neurotoxic damage. Toxicol. Appl. Pharmacol. 104:17-24

## Claims

1. Compound comprising at least one thioredoxin-like fold domain for use as medicament.

2. Compound comprising at least one thioredoxin-like fold domain for use for treating and/or the preventing degenerative disorders.

3. Compound according to claim 1 or 2 wherein it is chosen from peptides and proteins.

4. Compound according to any of claims 1 to 3, wherein it is chosen from selenoproteins and their fragments, preferably human selenoproteins and their fragments.

5. Compound according to any of claims 1 to 4, wherein it comprises a thioredoxin-like fold domain of formula CX₁X₂U, wherein X₁ is chosen from glycine, threonine and valine, and X₂ is chosen from alanine, leucine and serine.

6. Compound according to any of claims 1 to 5, wherein it comprises a thioredoxin-like fold domain chosen from CGAU (SEQ ID NO:1), CGLU (SEQ ID NO:2), CTSU (SEQ ID NO:3) and CVSU (SEQ ID NO:4).

7. Compound according to any of claims 1 to 6, wherein it is chosen from selenoprotein W, selenoprotein V, selenoprotein H, selenoprotein T and their fragments.

8. Compound according to any of claims 1 to 7, wherein it is chosen from human selenoprotein W, human selenoprotein V, human selenoprotein H, human selenoprotein T and their fragments.

9. Compound according to any of claims 1 to 8, wherein it is chosen from human selenoprotein T and its fragments.

10. Compound according to any of claims 1 to 9, wherein it is chosen from CVSU (SEQ ID NO:4), ICVSU (SEQ ID NO:5), CVSUG (SEQ ID NO:6), QICVSU (SEQ ID NO:7), QICVSUG (SEQ ID NO:8), CVSUGY (SEQ ID NO:9), ICVSUGY (SEQ ID NO:10), QICVSUGY (SEQ ID NO:11), FQICVSU (SEQ ID NO:12), FQICVSUG (SEQ ID NO:13), FQICVSUGY (SEQ ID NO:14), CVSUGYR (SEQ ID NO:15), ICVSUGYR (SEQ ID NO:16), QICVSUGYR (SEQ ID NO:17), FQICVSUGYR (SEQ ID NO:18), KFQICVSU (SEQ ID NO:19), KFQICVSUG (SEQ ID NO:20), KFQICVSUGY (SEQ ID NO:21), KFQICVSUGYR (SEQ ID NO:22), CVSUGYRR (SEQ ID NO:23), ICVSUGYRR (SEQ ID NO:24), QICVSUGYRR (SEQ ID NO:25), FQICVSUGYRR (SEQ ID NO:26), KFQICVSUGYRR (SEQ ID NO:27) and (X)n-KFQICVSUGYRR-(X')n_{'} in which X and X' represent any aminoacid, and n and n' are integers between 1 and 1000.

11. Compound according to any of claims 1 to 10, wherein said degenerative disorders are chosen from Parkinson's disease, Alzheimer's disease, Huntington's disease, multiple sclerosis, stroke, motoneuron-associated diseases like Charcot disease, epileptic seizures and neurotoxicant-associated diseases.

12. Pharmaceutical composition comprising a compound as defined in any of claims 1 to 11 in a pharmaceutically acceptable excipient.

13. Pharmaceutical composition as defined in claim 12 for an oral, intravenous, mucosal, nasal or pulmonary administration.


SEQUENCE LISTING

<110>  UNIVERSITE DE ROUEN

<120>  COMPOUNDS WITH THIOREDOXIN-LIKE FOLD DOMAIN FOR THE TREATMENT OF
       DEGENERATIVE DISORDERS

<130>  BEP110370 EP

<160>  49

<170>  PatentIn version 3.5

<210>  1
<211>  4
<212>  PRT
<213>  Artificial

<220>
<223>  artificial sequences wherein X is selenocystein


<220>
<221>  misc_feature
<222>  (4)..(4)
<223>  Xaa can be any naturally occurring amino acid

<400>  1

Cys Gly Ala Xaa
1


<210>  2
<211>  4
<212>  PRT
<213>  Artificial

<220>
<223>  artificial sequences wherein X is selenocystein


<220>
<221>  misc_feature
<222>  (4)..(4)
<223>  Xaa can be any naturally occurring amino acid

<400>  2

Cys Gly Leu Xaa
1


<210>  3
<211>  4
<212>  PRT
<213>  Artificial

<220>
<223>  artificial sequences wherein X is selenocystein


<220>
<221>  misc_feature
<222>  (4)..(4)
<223>  Xaa can be any naturally occurring amino acid

<400>  3

Cys Thr Ser Xaa
1


<210> 4
<211> 4
<212> PRT
<213> Artificial

<220>
<223> artificial sequences wherein X is selenocystein


<220>
<221> misc_feature
<222> (4)..(4)
<223> Xaa can be any naturally occurring amino acid

<400> 4

Cys Val Ser Xaa
1


<210> 5
<211> 5
<212> PRT
<213> Artificial

<220>
<223> artificial sequences wherein X is selenocystein


<220>
<221> misc_feature
<222> (5)..(5)
<223> Xaa can be any naturally occurring amino acid

<400> 5

Ile Cys Val Ser Xaa
1               5


<210> 6
<211> 5
<212> PRT
<213> Artificial

<220>
<223> artificial sequences wherein X is selenocystein


<220>
<221> misc_feature
<222> (4)..(4)
<223> Xaa can be any naturally occurring amino acid

<400> 6

Cys Val Ser Xaa Gly
1               5


<210> 7
<211> 6
<212> PRT

<213>  Artificial

<220>
<223>  artificial sequences wherein X is selenocystein


<220>
<221>  misc_feature
<222>  (6)..(6)
<223>  Xaa can be any naturally occurring amino acid

<400>  7

Gln Ile Cys Val Ser Xaa
1               5


<210>  8
<211>  7
<212>  PRT
<213>  Artificial

<220>
<223>  artificial sequences wherein X is selenocystein


<220>
<221>  misc_feature
<222>  (6)..(6)
<223>  Xaa can be any naturally occurring amino acid

<400>  8

Gln Ile Cys Val Ser Xaa Gly
1               5


<210>  9
<211>  6
<212>  PRT
<213>  Artificial

<220>
<223>  artificial sequences wherein X is selenocystein


<220>
<221>  misc_feature
<222>  (4)..(4)
<223>  Xaa can be any naturally occurring amino acid

<400>  9

Cys Val Ser Xaa Gly Tyr
1               5


<210>  10
<211>  7
<212>  PRT
<213>  Artificial

<220>
<223>  artificial sequences wherein X is selenocystein


<220>
<221>  misc_feature

<222>  (5)..(5)
<223>  Xaa can be any naturally occurring amino acid

<400>  10

Ile Cys Val Ser Xaa Gly Tyr
1               5


<210>  11
<211>  8
<212>  PRT
<213>  Artificial

<220>
<223>  artificial sequences wherein X is selenocystein


<220>
<221>  misc_feature
<222>  (6)..(6)
<223>  Xaa can be any naturally occurring amino acid

<400>  11

Gln Ile Cys Val Ser Xaa Gly Tyr
1               5


<210>  12
<211>  7
<212>  PRT
<213>  Artificial

<220>
<223>  artificial sequences wherein X is selenocystein


<220>
<221>  misc_feature
<222>  (7)..(7)
<223>  Xaa can be any naturally occurring amino acid

<400>  12

Phe Gln Ile Cys Val Ser Xaa
1               5


<210>  13
<211>  8
<212>  PRT
<213>  Artificial

<220>
<223>  artificial sequences wherein X is selenocystein


<220>
<221>  misc_feature
<222>  (7)..(7)
<223>  Xaa can be any naturally occurring amino acid

<400>  13

Phe Gln Ile Cys Val Ser Xaa Gly
1               5

<210>  14
<211>  9
<212>  PRT
<213>  Artificial

<220>
<223>  artificial sequences wherein X is selenocystein


<220>
<221>  misc_feature
<222>  (7)..(7)
<223>  Xaa can be any naturally occurring amino acid

<400>  14

Phe Gln Ile Cys Val Ser Xaa Gly Tyr
1               5


<210>  15
<211>  7
<212>  PRT
<213>  Artificial

<220>
<223>  artificial sequences wherein X is selenocystein


<220>
<221>  misc_feature
<222>  (4)..(4)
<223>  Xaa can be any naturally occurring amino acid

<400>  15

Cys Val Ser Xaa Gly Tyr Arg
1               5


<210>  16
<211>  8
<212>  PRT
<213>  Artificial

<220>
<223>  artificial sequences wherein X is selenocystein


<220>
<221>  misc_feature
<222>  (5)..(5)
<223>  Xaa can be any naturally occurring amino acid

<400>  16

Ile Cys Val Ser Xaa Gly Tyr Arg
1               5


<210>  17
<211>  9
<212>  PRT
<213>  Artificial

<220>
<223>  artificial sequences wherein X is selenocystein

<220>
<221>  misc_feature
<222>  (6)..(6)
<223>  Xaa can be any naturally occurring amino acid

<400>  17

Gln Ile Cys Val Ser Xaa Gly Tyr Arg
1               5


<210>  18
<211>  10
<212>  PRT
<213>  Artificial

<220>
<223>  artificial sequences wherein X is selenocystein


<220>
<221>  misc_feature
<222>  (7)..(7)
<223>  Xaa can be any naturally occurring amino acid

<400>  18

Phe Gln Ile Cys Val Ser Xaa Gly Tyr Arg
1               5                   10


<210>  19
<211>  8
<212>  PRT
<213>  Artificial

<220>
<223>  artificial sequences wherein X is selenocystein


<220>
<221>  misc_feature
<222>  (8)..(8)
<223>  Xaa can be any naturally occurring amino acid

<400>  19

Lys Phe Gln Ile Cys Val Ser Xaa
1               5


<210>  20
<211>  9
<212>  PRT
<213>  Artificial

<220>
<223>  artificial sequences wherein X is selenocystein


<220>
<221>  misc_feature
<222>  (8)..(8)
<223>  Xaa can be any naturally occurring amino acid

<400>  20

Lys Phe Gln Ile Cys Val Ser Xaa Gly
1               5

<210>  21
<211>  10
<212>  PRT
<213>  Artificial

<220>
<223>  artificial sequences wherein X is selenocystein

<220>
<221>  misc_feature
<222>  (8)..(8)
<223>  Xaa can be any naturally occurring amino acid

<400>  21

Lys Phe Gln Ile Cys Val Ser Xaa Gly Tyr
1               5                   10

<210>  22
<211>  11
<212>  PRT
<213>  Artificial

<220>
<223>  artificial sequences wherein X is selenocystein

<220>
<221>  misc_feature
<222>  (8)..(8)
<223>  Xaa can be any naturally occurring amino acid

<400>  22

Lys Phe Gln Ile Cys Val Ser Xaa Gly Tyr Arg
1               5                   10

<210>  23
<211>  8
<212>  PRT
<213>  Artificial

<220>
<223>  artificial sequences wherein X is selenocystein

<220>
<221>  misc_feature
<222>  (4)..(4)
<223>  Xaa can be any naturally occurring amino acid

<400>  23

Cys Val Ser Xaa Gly Tyr Arg Arg
1               5

<210>  24
<211>  9
<212>  PRT

<213>  Artificial

<220>
<223>  artificial sequences wherein X is selenocystein


<220>
<221>  misc_feature
<222>  (5)..(5)
<223>  Xaa can be any naturally occurring amino acid

<400>  24

Ile Cys Val Ser Xaa Gly Tyr Arg Arg
1               5


<210>  25
<211>  10
<212>  PRT
<213>  Artificial

<220>
<223>  artificial sequences wherein X is selenocystein


<220>
<221>  misc_feature
<222>  (6)..(6)
<223>  Xaa can be any naturally occurring amino acid

<400>  25

Gln Ile Cys Val Ser Xaa Gly Tyr Arg Arg
1               5                   10


<210>  26
<211>  11
<212>  PRT
<213>  Artificial

<220>
<223>  artificial sequences wherein X is selenocystein


<220>
<221>  misc_feature
<222>  (7)..(7)
<223>  Xaa can be any naturally occurring amino acid

<400>  26

Phe Gln Ile Cys Val Ser Xaa Gly Tyr Arg Arg
1               5                   10


<210>  27
<211>  12
<212>  PRT
<213>  Artificial

<220>
<223>  artificial sequences wherein X is selenocystein


<220>
<221>  misc_feature

<222>  (8)..(8)
<223>  Xaa can be any naturally occurring amino acid

<400>  27

Lys Phe Gln Ile Cys Val Ser Xaa Gly Tyr Arg Arg
1               5                   10


<210>  28
<211>  33
<212>  DNA
<213>  Artificial

<220>
<223>  artificial sequences

<400>  28
ggctttatgt aagcagttct aaactgtttc tgc                                    33


<210>  29
<211>  34
<212>  DNA
<213>  Artificial

<220>
<223>  artificial sequences

<400>  29
cgccccattt tataaacttt gtatgtttat gccc                                   34


<210>  30
<211>  25
<212>  DNA
<213>  Artificial

<220>
<223>  artificial sequences

<400>  30
atcgccaggc gttttctgag catac                                             25


<210>  31
<211>  25
<212>  DNA
<213>  Artificial

<220>
<223>  artificial sequences

<400>  31
gccagattac gtatatcctg gcagc                                             25


<210>  32
<211>  33
<212>  DNA
<213>  Artificial

<220>
<223>  artificial sequences

<400>  32
ggctttatgt aagcagttct aaactgtttc tgc                                    33

<210>  33
<211>  28
<212>  DNA
<213>  Artificial

<220>
<223>  artificial sequences

<400>  33
gcctaggttt tacctgagaa accaaagg                                          28


<210>  34
<211>  26
<212>  DNA
<213>  Artificial

<220>
<223>  artificial sequences

<400>  34
gaattcgcca gcatggaaaa ctgtct                                            26


<210>  35
<211>  28
<212>  DNA
<213>  Artificial

<220>
<223>  artificial sequences

<400>  35
ctcgaggggc tgcagtctcg gtctgaag                                          28


<210>  36
<211>  47
<212>  DNA
<213>  Artificial

<220>
<223>  artificial sequences

<400>  36
agcggctgtc tcaattaaga tgtctgccaa cctgggcggc gtgccca                     47


<210>  37
<211>  47
<212>  DNA
<213>  Artificial

<220>
<223>  artificial sequences

<400>  37
tgggcacgcc gcccaggttg gcagacatct taattgagac agccgct                     47


<210>  38
<211>  39
<212>  DNA
<213>  Artificial

<220>
<223>  artificial sequences

<400>  38
tttcagattt gtgtatcctc agggtacagg cgggtgttt                              39


<210>  39
<211>  39
<212>  DNA
<213>  Artificial

<220>
<223>  artificial sequences

<400>  39
aaacacccgc ctgtaccctg aggatacaca aatctgaaa                              39


<210>  40
<211>  25
<212>  RNA
<213>  Artificial

<220>
<223>  artificial sequences

<400>  40
acauagcauu ucuccuguca gucuu                                             25


<210>  41
<211>  25
<212>  RNA
<213>  Artificial

<220>
<223>  artificial sequences

<400>  41
aagacugaca ggaaagaugc uaugu                                             25


<210>  42
<211>  24
<212>  DNA
<213>  Artificial

<220>
<223>  artificial sequences

<400>  42
ggtctaagct ggaatctgga catc                                              24


<210>  43
<211>  24
<212>  DNA
<213>  Artificial

<220>
<223>  artificial sequences

<400>  43
tgcacattga gtttcatttc attg                                              24


<210>  44
<211>  17
<212>  DNA

<213>  Artificial

<220>
<223>  artificial sequences

<400>  44
tccggacgca ccctcat                                                    17


<210>  45
<211>  20
<212>  DNA
<213>  Artificial

<220>
<223>  artificial sequences

<400>  45
cggttgacct ccaggaaatc                                                 20


<210>  46
<211>  19
<212>  DNA
<213>  Artificial

<220>
<223>  artificial sequences

<400>  46
tagaggcagg gtcctgaag                                                  19


<210>  47
<211>  20
<212>  DNA
<213>  Artificial

<220>
<223>  artificial sequences

<400>  47
aatccatctc tggcctgact                                                 20


<210>  48
<211>  23
<212>  DNA
<213>  Artificial

<220>
<223>  artificial sequences

<400>  48
gaaaggatgg tcaatgacgc cga                                             23


<210>  49
<211>  21
<212>  DNA
<213>  Artificial

<220>
<223>  artificial sequences

<400>  49
gtcttcaatg tctgcatcct g                                               21